# EUROPÄISCHE PATENTSCHRIFT

(11) **EP 1 674 864 B1**
(45) Veröffentlichungstag und Bekanntmachung des Hinweises auf die Patenterteilung: **14.09.2016**
(21) Anmeldenummer: 05022888.1
(22) Anmeldetag: 20.10.2005
(51) Int. Cl.: G01N 33/18, G01N 31/00, G01N 31/12

(54) **Vorrichtung zum Analysieren von Flüssigkeiten**
Liquid analysis apparatus
Dispositif destiné à l'analyse de liquides

(30) Priorität: 09.12.2004 DE 102004059442
(43) Veröffentlichungstag der Anmeldung: 28.06.2006
(73) Patentinhaber: SICK AG, 79183 Waldkirch/Breisgau (DE)
(72) Erfinder: Heuer, Helge, 22397 Hamburg (DE); Kumm, Axel, 21079 Hamburg (DE); Saes, Thomas, 23881 Niendorf (DE)
(74) Vertreter: Manitz, Finsterwald & Partner GbR

(56) Entgegenhaltungen:
- EP-A2- 0 057 390
- DE-A1- 3 223 167
- DE-A1- 19 947 940
- DE-U1- 9 408 536
- US-A- 6 007 777
- US-A- 6 063 638

## Beschreibung

Die Erfindung betrifft eine Vorrichtung zum Analysieren von Flüssigkeiten mit einem Reaktor zum Umwandeln eines in einer Messflüssigkeit enthaltenen Stoffes in ein hinsichtlich des Stoffgehaltes analysierbares Messgas und einer Einrichtung zum Trennen des Messgases von einer dampf- oder gasförmigen Phase der Messflüssigkeit.

Derartige Analysevorrichtungen sind grundsätzlich bekannt und dienen insbesondere zum Bestimmen des Gesamtgehaltes an organischem Kohlenstoff (TOC). Dabei werden in dem Reaktor organische Kohlenstoffverbindungen durch Bestrahlen der Messflüssigkeit mit UV-Strahlung oxidiert und das dadurch entstehende Kohlendioxid als Messgas einem nachgeordneten Gasanalysator zugeführt, und zwar nachdem das Kohlendioxid von einer dampf- bzw. gasförmigen Phase der Messflüssigkeit getrennt wurde.

Problematisch bei bekannten Einrichtungen sind deren großer Platzbedarf und die hohen Herstellungskosten, die vor allem durch den diskreten Aufbau mit vergleichsweise weit voneinander entfernt liegenden Baugruppen bedingt sind. Dieser diskrete Aufbau ist im Wesentlichen eine Folge der für die gewünschte Analyse grundsätzlich erforderlichen Mehrzahl von einzelnen Prozessschritten. Bislang wird für jeden dieser Prozessschritte eine spezielle Baugruppe vorgesehen, wie dies beispielsweise bei den in der US 6,007,777, der DE 199 47 940 A1 und in der DE 32 23 167 A1 beschriebenen Anlagen der Fall ist.

Für die TOC-Bestimmung in Wasser beispielsweise müssen die organischen Kohlenstoffverbindungen zu Kohlendioxid oxidiert und anschließend in Form eines Gasstromes einem Kohlendioxid-Gasanalysator zugeführt werden. Für diese Analyse muss das Kohlendioxid zuvor aufbereitet und getrocknet werden. Des Weiteren müssen spezielle Maßnahmen zur Entsorgung des umgewandelten Messwassers getroffen werden, was derzeit mittels einer separaten Einrichtung zur Kondensatentsorgung erfolgt. Weiter verkompliziert wird die Angelegenheit dadurch, dass bei Verwendung von UV-Strahlung zur Erzeugung des Kohlendioxids sichergestellt werden muss, dass außerhalb des Reaktionsraumes kein Ozon erzeugt wird. Die hierfür erforderlichen Maßnahmen zur Abschirmung der UV-Strahlung, beispielsweise entsprechende Abdeckungen, ermöglichen keine unmittelbare visuelle Kontrolle des Reaktionsprozesses während des Analysebetriebs.

Aufgabe der Erfindung ist es daher, eine Analysevorrichtung der eingangs genannten Art zu schaffen, die einen sicheren und zuverlässigen Analysebetrieb gewährleistet und dabei möglichst einfach und kompakt aufgebaut ist, wobei insbesondere eine Sichtkontrolle des Reaktionsraumes während des laufenden Analysebetriebs möglich, eine einfache Zerlegbarkeit zu Wartungs- und Reinigungszwecken gewährleistet sowie sichergestellt sein soll, dass kein Ozon außerhalb des Reaktionsraumes erzeugt wird.

Die Lösung dieser Aufgabe erfolgt durch die Merkmale des Anspruchs 1 und insbesondere dadurch, dass der oben offene Reaktor während des Analysebetriebs unterhalb der Trenneinrichtung angeordnet ist und eine Einrichtung zur Entsorgung der unter Schwerkrafteinfluss zurück in den Reaktor gelangenden abgetrennten Flüssigkeit aufweist.

Gemäß der Erfindung umfasst der Reaktor ein Innenteil und ein das Innenteil zur Bildung eines Reaktionsraumes umgebendes Außenteil, wobei das Innenteil zur Aufnahme einer Strahlungsquelle ausgebildet ist, wohingegen das Außenteil als Abschirmung für die von der Strahlungsquelle stammende Strahlung ausgebildet ist.

Erfindungsgemäß ist der Reaktor, in welchem die Umwandlung beispielsweise von organischen Kohlenstoffverbindungen in Kohlendioxid erfolgt, mit seiner oben offenen Seite unterhalb der Trenneinrichtung angeordnet.

Die an der Trenneinrichtung anfallende Flüssigkeit kann so in den Reaktor zurückfallen und über den Reaktor entsorgt werden. Hierzu ist erfindungsgemäß der Reaktor mit einer entsprechenden Entsorgungseinrichtung versehen.

Indem auf diese Weise der Reaktor einerseits und die Trenneinrichtung andererseits zu einer kompakten Funktionseinheit zusammengeschlossen werden, kann nicht nur auf eine separate Einrichtung zur Kondensatentsorgung verzichtet werden, sondern es ergibt sich darüber hinaus insgesamt ein einfacher und platzsparender Aufbau der gesamten Analysevorrichtung. Es hat sich herausgestellt, dass durch das erfindungsgemäße Zusammenfassen von Reaktor und Trenneinrichtung eine wirksame und betriebssichere Trennung des zu analysierenden, durch die Umwandlung im Reaktor erzeugten Messgases von der Messflüssigkeit erreicht werden kann.

Ein Gasanalysator kann folglich der Trenneinrichtung nachgeordnet werden, ohne dass spezielle Schutzmaßnahmen für den Gasanalysator erforderlich wären. Hierdurch kann die Platzierung des Gasanalysators ohne störende Randbedingungen und insbesondere ohne Rücksicht auf separate Schutzeinrichtungen praktisch ausschließlich im Hinblick auf eine Optimierung der gesamten Analysevorrichtung im Sinne eines einfachen und kompakten Aufbaus erfolgen.

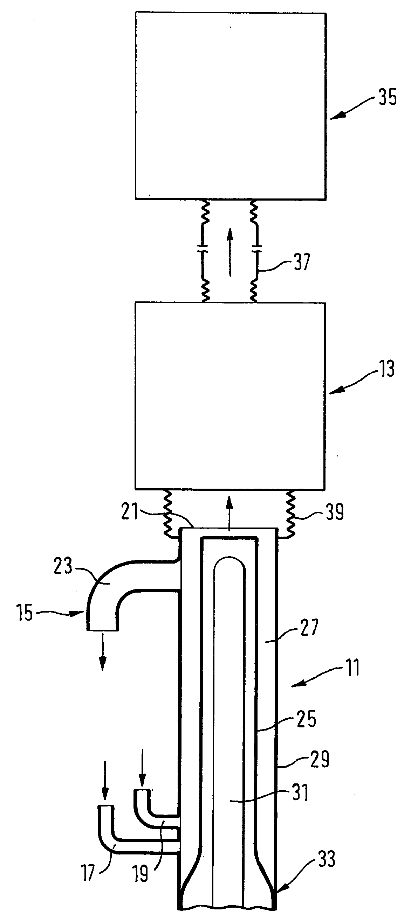

Bevorzugte Ausführungsformen der Erfindung sind auch in den abhängigen Ansprüchen, der Beschreibung sowie der Zeichnung angegeben.

Bei der Trenneinrichtung handelt es sich insbesondere um eine Kühleinrichtung, insbesondere eine Kühlfalle, einen Messgaskühler oder eine Kondensationsstrecke. Die durch die Kühleinrichtung bewirkte Trocknung des Messgases kann beispielsweise nach dem Prinzip einer Vortex-Kühlung erfolgen.

Insbesondere ist die erfindungsgemäße Analysevorrichtung für einen kontinuierlichen Analysebetrieb ausgebildet. Dies bedeutet, dass die Messflüssigkeit dem Reaktor kontinuierlich zugeführt wird, wodurch zum einen ein stetiger zu analysierender Messgasstrom entsteht und zum anderen eine permanente Entsorgung der abgetrennten Messflüssigkeit bzw. des Kondensats erfolgt.

Vorzugsweise besitzt der Reaktor zwischen zumindest einem unteren Einlass für die den Stoff enthaltende Messflüssigkeit einerseits und seiner oberen Öffnung andererseits wenigstens einen Auslass zur Entsorgung der abgetrennten Flüssigkeit. Bei einem kontinuierlichen Analysebetrieb, d.h. bei kontinuierlicher Zufuhr der Messflüssigkeit in den Reaktor, entsteht in der Regel pro Zeiteinheit weniger Kondensat als Messflüssigkeit nachgefördert wird. Es kommt folglich zu einem so genannten Messflüssigkeitsüberschuss, der entsorgt werden muss.

Gemäß einem bevorzugten Ausführungsbeispiel der Erfindung ist ein Auslass des Reaktors ein gemeinsamer Auslass für die abgetrennte Flüssigkeit bzw. das Kondensat einerseits und für überschüssige Messflüssigkeit bzw. Messfüssigkeitsüberschuss andererseits. In vorteilhafter Weise kann ein für einen kontinuierlichen Analysebetrieb ohnehin zur Entsorgung des Überschusses erforderlicher Auslass gleichzeitig zur Entsorgung der von der Trenneinrichtung stammenden Flüssigkeit genutzt werden.

Der Auslass kann nach Art eines Überlaufs und/oder Syphons ausgebildet sein.

Reaktoren mit innenliegender Strahlungsquelle und äußerer Umhüllung zur Bildung eines Reaktionsraumes für eine Messflüssigkeit sind grundsätzlich bekannt. Beispielsweise in Wasser enthaltene organische Kohlenstoffverbindungen lassen sich durch UV-Bestrahlung in Kohlendioxid und damit in ein analysierbares Messgas umwandeln. Als UV-Strahlungsquelle dienen insbesondere Quecksilber-Niederdrucklampen. Der besondere Vorteil des erfindungsgemäßen Ausführungsbeispiels besteht darin, dass durch die Ausbildung des Außenteils als Abschirmung für die Strahlung der Reaktor sozusagen selbst für die erforderliche Strahlensicherheit sorgt und somit keine separaten Schutzmaßnahmen, z.B. in Form von störenden Abdeckungen, erforderlich sind.

In einer bevorzugten Ausgestaltung des erfindungsgemäßen Reaktors ist das Außenteil aus einem gefärbten oder gebeizten Glasmaterial hergestellt. Bei dem Glasmaterial handelt es sich insbesondere um ein Borosilikatglas.

Mit einem derartigen Reaktoraußenteil kann nicht nur die erforderliche Strahlensicherheit gewährleistet, sondern gleichzeitig eine Sichtkontrolle des Reaktionsraumes ermöglicht werden. Es hat sich herausgestellt, dass sich das Außenteil des Reaktors derart herstellen lässt, dass es für Strahlung im für das menschliche Auge sichtbaren Bereich durchlässig ist und somit eine Sichtkontrolle des Reaktionsraumes ermöglicht, ohne dass die Strahlenschutzfunktion beeinträchtigt wird. Des Weiteren kann ein solches Außenteil vergleichsweise preisgünstig hergestellt werden.

Gemäß einem weiteren bevorzugten Ausführungsbeispiel der Erfindung ist vorgesehen, dass das Innenteil und das Außenteil aus einem Glasmaterial hergestellt und insbesondere zur bodenseitigen Abdichtung des Reaktionsraumes mit einem Dichtschliff versehen sind.

Eine Abdichtung des Reaktionsraumes kann einfach durch Zusammenfügen von Innenteil und Außenteil erfolgen, die hierzu mit einer entsprechenden Formgebung versehen sind.

Ein weiterer besonderer Vorteil des erfindungsgemäßen Reaktors ist dessen einfache Zerlegbarkeit, wodurch Wartungs- und Reinigungsaufgaben beträchtlich erleichtert werden.

Die Erfindung wird im Folgenden beispielhaft unter Bezugnahme auf die Zeichnung beschrieben, deren einzige Figur rein schematisch einen Teil einer erfindungsgemäßen Analysevorrichtung zeigt.

Die erfindungsgemäße Analysevorrichtung umfasst einen Reaktor 11, dem eine Trenneinrichtung in Form eines Kühlers 13 sowie ein Gasanalysator 35 nachgeordnet sind. Bei den Verbindungen 37, 39 zwischen den genannten Bestandteilen kann es sich um herkömmliche Schlauchverbindungen handeln, die eine Fluiddichtigkeit gegenüber der Umgebung gewährleisten. Während die Verbindung zwischen dem Kühler 13 und dem Analysator 35 prinzipiell eine beliebige Länge aufweisen kann, die eine optimale Platzierung des Analysators 35 innerhalb der Gesamtvorrichtung ermöglicht, handelt es sich bei der Verbindung 39 zwischen Reaktor 11 und Kühler 13 um eine sehr kurze, insbesondere nur wenige Zentimeter lange Strömungsstrecke.

Der Reaktor 11 umfasst ein Innenteil 25 in Form eines Tauchrohres aus Quarzglas, in welches bei zusammengesetztem Reaktor 11 eine als UV-Strahlungsquelle dienende Quecksilber-Niederdrucklampe 31 eingeschoben ist. Das Tauchrohr 25 ist von einem das Außenteil des Reaktors 11 bildenden, braun gebeizten Hüllrohr 29 umgeben, das im Bereich seines unteren Endes mit zwei Einlässen 17, 19 und im Bereich seines oberen Endes mit einem Auslass 23 versehen ist. In dem dargestellten Ausführungsbeispiel ist der Strömungsquerschnitt des Auslasses 23 deutlich größer als derjenige der beiden Einlässe 17, 19. Das Hüllrohr 29 ist oben offen.

Die beiden Glasrohre 25, 29 sind in einem Bereich, der bei zusammengesetztem Reaktor 11 unterhalb der Einlässe 17, 19 des eingefärbten Hüllrohres 29 liegt, mit einem Dichtschliff 33 dergestalt versehen, dass bei in das Hüllrohr 29 eingeschobenem Tauchrohr 25 ein von den beiden Rohren 25, 29 begrenzter ringförmiger Reaktionsraum 27 des Reaktors 11 nach unten fluiddicht abgedichtet ist. Ein entsprechender Formschluss zwischen den beiden Rohren 25, 29 kann beispielsweise durch eine konusartige Formgebung der beiden unteren Rohrenden erreicht werden. In der Figur ist die Abdichtung rein schematisch durch eine Verbreiterung lediglich des inneren Tauchrohres 25 an dessen unterem Ende angedeutet.

Der Reaktionsraum 27 des Reaktors 11 ist derart bemessen, dass die während des Analysebetriebs innerhalb des Reaktors 11 befindliche Menge an Messflüssigkeit etwa bei 20 ml liegt. Nach oben ist der Reaktionsraum 27 aufgrund der Ausgestaltung des Hüllrohres 29 offen, so dass im Reaktionsraum 27 entstehendes Gas aus der oberen Reaktoröffnung 21 austreten und zu dem nachgeordneten Kühler 13 gelangen kann.

Bei dem unmittelbar oberhalb der Austrittsöffnung 21 des Reaktors 11 angeordneten Kühler 13 handelt es sich um eine Kühlfalle z.B. in Form eines Messgaskühlers oder einer Kondensationsstrecke.

Zusammen mit weiteren, nicht dargestellten Komponenten kann die in der Figur dargestellte Baugruppe der erfindungsgemäßen Analysevorrichtung in einem Gehäuse z.B. in Form eines Schaltschrankes mit relativ kleinen Abmessungen untergebracht werden. Die erfindungsgemäße Analysevorrichtung ist hierdurch zu einer kompakten Gesamteinheit zusammengefasst.

Insbesondere dient die erfindungsgemäße Analysevorrichtung zur Bestimmung des Gesamtgehaltes an organischem Kohlenstoff (TOC = Total Organic Carbon) in Wasser. Das zu untersuchende Messwasser wird dem Reaktor über einen der beiden unteren Einlässe 17, 19 zugeführt. Der andere Einlass 19, 17 dient zur Zufuhr von Luft. Durch Bestrahlung des im Reaktionsraum 27 zwischen innerem Tauchrohr 25 und äußerem Hüllrohr 29 befindlichen Messwassers mit UV-Strahlung, die von der in das Tauchrohr 25 eingeschobenen Quecksilber-Niederdrucklampe 31 erzeugt wird, werden die im Messwasser enthaltenen organischen Kohlenstoffverbindungen durch Oxidation in Kohlendioxid umgewandelt.

Das Kohlendioxid tritt zusammen mit einer dampf- bzw. gasförmigen Phase des Messwassers aus dem Reaktor 11 durch die obere Öffnung 21 des Hüllrohres 29 bzw. Reaktionsraumes 27 aus und gelangt unmittelbar zur Kühlfalle 13. Das dadurch kondensierende Messwasser fällt schwerkraftbedingt zurück in den Reaktionsraum 27 und wird zusammen mit dem durch die kontinuierliche Zufuhr bedingten Messwasserüberschuss über den als Syphon wirkenden Auslass 23 des Reaktors 11 entsorgt.

Das mittels der Kühlfalle 13 getrocknete Kohlendioxid gelangt über die Verbindung 37 zum Gasanalysator 35. Der erfindungsgemäße Reaktor 11 ist derart ausgelegt, dass die im Messwasser enthaltenen Kohlenstoffverbindungen vollständig oxidiert werden, so dass mittels des Gasanalysators 35 die gewünschte TOC-Bestimmung möglich ist.

Aufgrund der Einfärbung des Hüllrohres 29 kann eine Bedienungsperson mit ungeschützten Augen eine visuelle Sichtkontrolle des Reaktionsraumes 27 während des laufenden Analysebetriebs durchführen und insbesondere eventuelle Verschmutzungen des Reaktors 11 sofort erkennen. Insbesondere kann der Reaktor 11 mit einem Blick auf das eine ordnungsgemäße Funktion signalisierende Vorhandensein von aufsteigenden Luftblasen im Reaktionsraum 27 überprüft werden. Die Erzeugung von Ozon außerhalb des Reaktors 11 wird durch die UV-Abschirmung des Hüllrohres 29 sicher verhindert. Wartungs- und Reinigungsarbeiten am Reaktor 11 können wegen der einfachen Zerlegbarkeit schnell und problemlos durchgeführt werden.

Bei den vorstehend erwähnten, nicht dargestellten Komponenten handelt es sich insbesondere um eine Mehrkanal-Dosierpumpe, einen Verteiler oder Mischer, einen Stripper, einen Phasentrenner und ein Absorptionsgefäß oder Filter. Diese dem Reaktor 11 vorgelagerten Komponenten dienen zur Auf- oder Vorbereitung des zu analysierenden Probenwassers. Insbesondere soll sichergestellt werden, dass das in den Reaktor 11 gelangende Messwasser frei von anorganischen Kohlenstoffbestandteilen ist und die in den Reaktor 11 gelangende Luft kein Kohlendioxid enthält, um eine möglichst genaue TOC-Bestimmung zu erzielen.

Das Probenwasser, das gegebenenfalls mit Zusätzen wie z.B. Oxidationsmittel und Säure versehen wird, gelangt über die Pumpe in den Verteiler oder Mischer, dem außerdem auch als Trägergas bezeichnete Luft zugeführt wird, und zwar bevorzugt mittels einer in den Analysator 35 integrierten Pumpe, damit die Luftzufuhr in Abhängigkeit von Parametern der Messgasanalyse gesteuert oder geregelt werden kann.

Vom Verteiler oder Mischer gelangen das Probenwasser und die Luft in den Phasentrenner, und zwar über den zwischen Pumpe und Phasentrenner angeordneten Stripper, der für eine optimale Vermengung von Probenwasser und Luft sorgt. Eine Besonderheit ist die äußerst einfache und kostengünstige Ausgestaltung des Strippers, der aus einem relativ langen Teflonrohr mit einer Länge von z.B. 1,6 m besteht, das einfach - um Platz zu sparen - auf eine Rolle oder Trommel gewickelt ist. Es hat sich herausgestellt, dass ein derartiges Rohr eine ausreichend lange Reaktions- oder Kontaktstrecke bereitstellen kann, um eine optimale Vermengung zu erreichen. Bislang wurden derartige Stripper in Form von relativ aufwändigen und teuren Glasgefäßen mit insbesondere kaskadenartigen Einrichtungen verwendet. In dem Phasentrenner wird das vom Stripper kommende Gemenge in eine flüssige und eine gasförmige Phase getrennt.

Das Probenwasser ist aufgrund dieser Aufbereitung frei von anorganischen Kohlenstoffbestandteilen und wird über einen weiteren Kanal der Pumpe einem der beiden Einlässe 17, 19 des Reaktors 11 als Messwasser zugeführt. Die aus dem Phasentrenner austretende Luft wird dem anderen Einlass 19, 17 des Reaktors 11 über das Absorptionsgefäß bzw. Filter zugeführt, das die Luft von Kohlendioxid befreit.

### Bezugszeichenliste

- 11: Reaktor
- 13: Trenneinrichtung, Kühler
- 15: Entsorgungseinrichtung
- 17: Einlass
- 19: Einlass
- 21: obere Öffnung des Reaktors
- 23: Auslass
- 25: Innenteil, Tauchrohr
- 27: Reaktionsraum
- 29: Außenteil, Hüllrohr
- 31: Strahlungsquelle, Quecksilber-Niederdrucklampe
- 33: Dichtschliff
- 35: Analysator
- 37: Verbindung
- 39: Verbindung

## Patentansprüche

1. Vorrichtung zum Analysieren von Flüssigkeiten, insbesondere zum Bestimmen des Gesamtgehaltes an organischem Kohlenstoff (TOC), mit
- einem Reaktor (11), welcher zum Umwandeln eines in einer Messflüssigkeit enthaltenen Stoffes in ein hinsichtlich des Stoffgehaltes analysierbares Messgas eine Strahlungsquelle (31) umfasst,
- und einer Einrichtung (13) zum Trennen des Messgases von einer dampf- oder gasförmigen Phase der Messflüssigkeit, wobei der oben offene Reaktor (11) während des Analysebetriebs unterhalb der Trenneinrichtung (13) angeordnet ist, und Reaktor (11) und Trenneinrichtung (13) so ausgebildet sind, dass abgetrennte Messflüssigkeit unter Schwerkrafteinfluss zurück in den Reaktor (11) gelangen kann, und der Reaktor (11) eine Einrichtung (15) zur Entsorgung der auf diese Weise zurück in den Reaktor (11) gelangten Messflüssigkeit aufweist; wobei der Reaktor (11) ein Innenteil (25) und ein das Innenteil (25) zur Bildung eines Reaktionsraumes (27) umgebendes Außenteil (29) umfasst, wobei das Innenteil (25) die Strahlungsquelle (31), insbesondere eine UV-Strahlungsquelle, zum Bestrahlen der im Reaktionsraum (27) befindlichen Messflüssigkeit aufnimmt und das Außenteil (29) als Abschirmung für die von der Strahlungsquelle (31) stammende Strahlung ausgebildet ist.

2. Vorrichtung nach Anspruch 1,
**dadurch gekennzeichnet,**
**dass** die Trenneinrichtung (13) eine Kühleinrichtung ist.

3. Vorrichtung nach Anspruch 1 oder 2,
**dadurch gekennzeichnet,**
**dass** sie für einen kontinuierlichen Analysebetrieb ausgebildet ist.

4. Vorrichtung nach einem der vorhergehenden Ansprüche,
**dadurch gekennzeichnet ,**
**dass** der Reaktor (11) zwischen zumindest einem unteren Einlass (17, 19) für die den Stoff enthaltende Messflüssigkeit und seiner oberen Öffnung (21) wenigstens einen Auslass (23) zur Entsorgung der abgetrennten Messflüssigkeit aufweist.

5. Vorrichtung nach einem der vorhergehenden Ansprüche,
**dadurch gekennzeichnet,**
**dass** ein Auslass (23) des Reaktors (11) ein gemeinsamer Auslass für die abgetrennte und für überschüssige Messflüssigkeit ist.

6. Vorrichtung nach Anspruch 4 oder 5,
**dadurch gekennzeichnet,**
**dass** der Auslass (23) nach Art eines Überlaufs und/oder Syphons ausgebildet ist.

7. Vorrichtung nach einem der vorhergehenden Ansprüche,
**dadurch gekennzeichnet,**
**dass** das Außenteil (29) aus einem gefärbten oder gebeizten Glasmaterial hergestellt ist, insbesondere aus einem Borosilikatglas.

8. Vorrichtung nach einem der vorhergehenden Ansprüche,
**dadurch gekennzeichnet,**
**dass** das Außenteil (29) zur Ermöglichung einer Sichtkontrolle des Reaktionsraumes (27) für Strahlung im für das menschliche Auge sichtbaren Bereich durchlässig ausgebildet ist.

9. Vorrichtung nach einem der vorhergehenden Ansprüche,
**dadurch gekennzeichnet,**
**dass** das Innenteil (25) und das Außenteil (29) aus einem Glasmaterial hergestellt und insbesondere zur bodenseitigen Abdichtung des Reaktionsraumes (27) mit einem Dichtschliff (33) versehen sind.

## Claims

1. An apparatus for analyzing liquids,
in particular for determining the total organic carbon (TOC); comprising
- a reactor (11) which comprises a radiation source (31) for converting a substance included in a measurement liquid into a measurement gas which can be analyzed with respect to the substance content;
- and a device (13) for separating the measurement gas from a vapor phase or gaseous phase of the measurement liquid,
wherein the upwardly open reactor (11) is arranged beneath the separating device (13) during analysis operation, and the reactor (11) and the separating device (13) are configured such that separated measurement liquid can move back into the reactor (11) under the influence of gravity, and the reactor (11) has a device (15) for the disposal of the measurement liquid which has moved back into the reactor (11) in this manner; and
wherein the reactor (11) comprises an inner part (25) and an outer part (29) which surrounds the inner part (25) to form a reaction space (27), with the inner part (25) receiving the radiation source (31), in particular a UV radiation source, for irradiating the measurement liquid located in the reaction space (27) and the outer part (29) being configured as a screen for the radiation emanating from the radiation source (31).

2. An apparatus in accordance with claim 1,
**characterized in that**
the separating device (13) is a cooling device.

3. An apparatus in accordance with claim 1 or claim 2,
**characterized in that**
it is configured for continuous analysis operation.

4. An apparatus in accordance with any one of the preceding claims,
**characterized in that**
the reactor (11) has at least one outlet (23) for the disposal of the separated measurement fluid between at least one lower inlet (17, 19) for the measurement liquid including the substance and its upper opening (21).

5. An apparatus in accordance with any one of the preceding claims,
**characterized in that**
an outlet (23) of the reactor (11) is a common outlet for the separated measurement liquid and excess measurement liquid.

6. An apparatus in accordance with claim 4 or claim 5,
**characterized in that**
the outlet (23) is configured in the manner of an overflow and/or syphon.

7. An apparatus in accordance with any one of the preceding claims,
**characterized in that**
the outer part (29) is manufactured from a dyed or stained glass material, in particular from borosilicate glass.

8. An apparatus in accordance with any one of the preceding claims,
**characterized in that**
the outer part (29) is permeable for radiation in the range visible to the human eye to enable a visible inspection of the reaction space (27).

9. An apparatus in accordance with any one of the preceding claims,
**characterized in that**
the inner part (25) and the outer part (29) are manufactured from a glass material and are in particular provided with a ground sealing surface (33) for the base-side sealing of the reaction space (27).

## Revendications

1. Dispositif destiné à l'analyse de liquides,
en particulier à déterminer la teneur totale en carbone organique (TOC),
- un réacteur (11) qui comprend une source de rayonnement (31) pour transformer une substance contenue dans un liquide de mesure en un gaz de mesure analysable à l'égard de la teneur en substance,
et un moyen (13) destiné à séparer le gaz de mesure vis-à-vis d'une phase en vapeur ou gazeuse du liquide de mesure,
le réacteur (11) ouvert en haut étant agencé au-dessous du moyen de séparation (13) pendant l'analyse en cours,
et le réacteur (11) et le dispositif de séparation (13) sont réalisés de telle sorte que
le liquide de mesure séparé peut revenir dans le réacteur (11) sous l'action de la pesanteur et que le réacteur (11) comprend un moyen (15) destiné à évacuer le liquide de mesure revenu de cette manière dans le réacteur (11);
dans lequel
le réacteur (11) inclut une partie intérieure (25) et une partie extérieure (29) qui entoure la partie intérieure (25) pour former une chambre de réaction (27), la partie intérieure (25) recevant la source de rayonnement (31), en particulier une source de rayonnement UV, afin d'irradier le liquide de mesure situé dans la chambre de réaction (27), et la partie extérieure (29) étant réalisée à titre de blindage pour le rayonnement émanant de la source de rayonnement (31).

2. Dispositif selon la revendication 1,
**caractérisé en ce que**
le dispositif de séparation (13) est un dispositif de refroidissement.

3. Dispositif selon la revendication 1 ou 2,
**caractérisé en ce qu'**il est conçu pour un fonctionnement d'analyse en continu.

4. Dispositif selon l'une des revendications précédentes,
**caractérisé en ce que**
le réacteur (11) présente, entre au moins une entrée inférieure (17, 19) pour le liquide de mesure contenant la substance et son ouverture supérieure (21), au moins une sortie (23) pour évacuer le liquide de mesure séparé.

5. Dispositif selon l'une des revendications précédentes,
**caractérisé en ce que**
une sortie (23) du réacteur (11) est une sortie commune pour le liquide de mesure séparé et pour le liquide de mesure excédentaire.

6. Dispositif selon la revendication 4 ou 5,
**caractérisé en ce que**
la sortie (23) est réalisée à la manière d'un trop-plein et/ou d'un siphon.

7. Dispositif selon l'une des revendications précédentes,
**caractérisé en ce que**
la partie extérieure (29) est réalisée en un matériau de verre coloré ou teinté, en particulier en verre borosilicate.

8. Dispositif selon l'une des revendications précédentes,
**caractérisé en ce que**
la partie extérieure (29) est réalisée perméable au rayonnement dans la zone visible pour l'oeil humain, afin de permettre un contrôle visuel de la chambre de réaction (27).

9. Dispositif selon l'une des revendications précédentes,
**caractérisé en ce que**
la partie intérieure (25) et la partie extérieure (29) sont réalisées en un matériau de verre et sont en particulier pourvues d'un polissage du type rodage à l'émeri (33) pour l'étanchement côté fond de la chambre de réaction (27).
